# EUROPEAN PATENT APPLICATION

(11) **EP 4 083 214 A1**
(43) Date of publication of application: **02.11.2022**
(21) Application number: 21808090.1
(22) Date of filing: 20.05.2021
(51) Int. Cl.: C12N 15/77, C12P 13/06, C07K 14/34

(54) **MICROORGANISM HAVING ENHANCED L-BRANCHED CHAIN AMINO ACID PRODUCTION ABILITY AND METHOD FOR PRODUCING L-BRANCHED CHAIN AMINO ACID BY USING SAME**

(30) Priority: 21.05.2020 KR 20200061175
(71) Applicant: CJ Cheiljedang Corporation, Seoul 04560 (KR)
(72) Inventor: YOON, Byoung Hoon, Seoul 04560 (KR); KIM, Seon Hye, Seoul 04560 (KR); CHANG, Jin Sook, Seoul 04560 (KR); KIM, Ju-yeon, Seoul 04560 (KR); CHOI, Sun Hyoung, Seoul 04560 (KR); KIM, Kyungrim, Seoul 04560 (KR); KIM, Ju Eun, Seoul 04560 (KR); KIM, Hyung Joon, Seoul 04560 (KR)
(74) Representative: Regimbeau
(86) International application number: PCT/KR2021/006262
(87) International publication number: WO 2021/235855

(57) **Abstract**

Disclosed is an L-branched-chain amino acid-producing microorganism having enhanced activity of regulator of acetate metabolism A and a method for producing an L-branched-chain amino acids using the same.

## Description

### [Technical Field]

The present disclosure relates to an L-branched-chain amino acid-producing microorganism having enhanced activity of regulator of acetate metabolism A and a method for producing an L-branched-chain amino acid using the same.

### [Background Art]

L-Amino acids, as basic structural units of proteins, are used as main ingredients for pharmaceuticals, food additives, animal feeds, nutritional supplements, pesticides, disinfectants, and the like. In particular, branched-chain amino acids (BCAAs), as a generic term for the essential amino acids L-valine, L-leucine, and L-isoleucine, are known for antioxidant effects and effects on directly promoting protein synthesis of muscle cells.

Meanwhile, production of branched-chain amino acids using microorganisms is mainly conducted by microorganisms of the genus *Escherichia* or microorganisms of the genus *Corynebacterium,* and it has been known that branched-chain amino acids are produced via biosynthesis using 2-ketoisocaproate as a precursor from pyruvic acid through various stages (US 10316297 B2, US 10526586 B2, and US 10072278 B2). However, production of L-branched-chain amino acids by using the microorganisms has a problem in that mass production is not easy industrially.

### [Disclosure]

### [Technical Problem]

With this background, as a result of intensive efforts to enhance the ability to produce L-branched-chain amino acids using microorganisms, the present inventors have found that the ability to produce L-branched-chain amino acids is significantly increased by increasing expression of the regulator of acetate metabolism A (hereinafter referred to as RamA) of the microorganisms, thereby completing the present disclosure.

### [Technical Solution]

An object of the present disclosure is to provide an L-branched-chain amino acid-producing microorganism having enhanced activity of regulator of acetate metabolism A.

Another object of the present disclosure is to provide an L-branched-chain amino acid-producing microorganism including a polynucleotide having promoter activity and including substitution of a nucleotide with a different nucleotide at one or more corresponding positions selected from positions of the 34^{th}, 36^{th}, 37^{th}, 41^{st}, and 43^{rd} nucleotides of a nucleotide sequence as set forth in SEQ ID NO: 1.

Another object of the present disclosure is to provide a method for producing an L-branched-chain amino acid, the method including culturing the microorganism in a culture medium.

Another object of the present disclosure is to provide a polynucleotide having promoter activity and including substitution of a nucleotide with a different nucleotide at one or more corresponding positions selected from positions 34, 36, 37, 41, and 43 of a nucleotide sequence as set forth in SEQ ID NO: 1.

### [Advantageous Effects]

L-Branched-chain amino acids may be produced with high yields by culturing the L-branched-chain amino acid-producing microorganism including the polynucleotide of the present disclosure.

Also, the amino acids produced by the present disclosure may be applied to a variety of products such as human foods, feed additives, or pharmaceuticals as well as animal feeds or animal feed additives.

### [Best Mode]

The present disclosure will be described in detail. Meanwhile, each description and embodiment disclosed in the present disclosure may be applied to different descriptions and embodiments herein. In addition, all combinations of various components disclosed in the present disclosure are included within the scope of the present disclosure. Furthermore, the scope of the present disclosure should not be limited by the descriptions provided below.

Those skilled in the art will recognize or be able to ascertain, using no more than routine experimentation, many equivalents to specific embodiments of the present disclosure. Such equivalents are intended to be encompassed in the scope of the following claims.

An aspect of the present disclosure provides an L-branched-chain amino acid-producing microorganism having enhanced activity of regulator of acetate metabolism A.

As used herein, the term "regulator of acetate metabolism A" refers to a regulatory protein related to the metabolism of acetic acid as a target protein of the present disclosure and may be encoded by *ramA* gene.

In the present disclosure, expression of the regulator of acetate metabolism A may be enhanced, and the enhancement of expression may result in an increase in the ability to produce an L-branched-chain amino acid.

As used herein, the term "enhancement" of the activity of the regulator of acetate metabolism A means that the activity of the regulator of acetate metabolism A is increased compared to intrinsic activity. The enhancement may be used interchangeably with activation, up-regulation, overexpression, increase, and the like. In this regard, the activation, enhancement, up-regulation, overexpression, and increase may include all of those exhibiting activity that was not originally possessed or exhibiting improved activity compared to intrinsic activity or activity before modification. The "intrinsic activity" refers to activity of a particular polypeptide originally possessed by a parent strain or non-modified microorganism before transformation when the microorganism is transformed by genetic modification caused by a natural or artificial factor. This term may be used interchangeably with "activity before modification". The "enhancement", "up-regulation", "overexpression", or "increase" of activity of a polypeptide compared to intrinsic activity means that the activity and/or concentration (expression level) of a particular polypeptide is improved compared to those originally possessed by a parent strain or non-modified microorganism before transformation.

The enhancement may be achieved by introducing a foreign polypeptide or by enhancing activity and/or increasing a concentration (expression level) of an endogenous polypeptide. Whether the activity of the regulator of acetate metabolism A is enhanced or not may be identified based on improvement/increase of the activity or expression level of the polypeptide or the amount of a product released from the polypeptide.

Enhancement of the activity of the regulator of acetate metabolism A may be achieved by applying various methods well known in the art and the methods are not limited as long as the activity of a target polypeptide is enhanced compared to that of the microorganism before modification. Specifically, any genetic engineering and/or protein engineering methods well known in the art as routine methods of molecular biology may be used, without limitation (for example, Sitnicka et al. Functional Analysis of Genes. Advances in Cell Biology. 2010, Vol. 2. 1-16, Sambrook et al. Molecular Cloning 2012).

Specifically, the enhancement of the activity of the regulator of acetate metabolism A may be achieved by:
1) increasing the copy number of a polynucleotide encoding the polypeptide in cells;
2) replacing a gene expression regulatory sequence on the chromosome encoding the polypeptide with a sequence improving expression of the polypeptide or introducing a modification thereinto;
3) modifying a nucleotide sequence encoding a start codon or a 5'-UTR region of a gene transcript encoding the polypeptide;
4) modifying an amino acid sequence of the polypeptide to enhance the activity of the polypeptide;
5) modifying a sequence of a polynucleotide encoding the polypeptide to enhance the activity of the polypeptide (e.g., modifying a nucleotide sequence of a gene of a polypeptide to encode a polypeptide modified to have enhanced activity);
6) introducing a foreign polypeptide having the activity of the polypeptide a foreign polynucleotide encoding the same;
7) optimizing a codon of a polynucleotide encoding the polypeptide;
8) modifying or chemically modifying an exposed region selected by analyzing a three-dimensional structure of the polypeptide; or
9) any combination of two or more selected from the 1) to 8) above, without being limited thereto.

### More specifically,

the increasing the copy number of a polynucleotide encoding the polypeptide described in 1) above may be achieved by introducing a vector, which replicates and functions irrespective of a host cell and is operatively linked to the polynucleotide encoding the polypeptide, into the host cell. Alternatively, the increase in the copy number may be achieved by introducing one copy or two or more copies of the polynucleotide encoding the polypeptide into the chromosome of the host cell. Introduction into the chromosome may be performed by introducing a vector capable of inserting the polynucleotide into the chromosome of the host cell into the host cell, without being limited thereto. The vector is as described below.

The replacing a gene expression regulatory sequence (or expression regulatory region) on the chromosome encoding the polypeptide with a sequence improving expression of the polypeptide or introducing a modification thereinto described in 2) above may be achieved, for example, by inducing mutation in the sequence by deletion, insertion, non-conservative, or conservative substitution, or any combination thereof, or by replacing the gene expression regulatory sequence with a sequence capable of improving expression of the polypeptide, in order to further enhance the activity of the expression regulatory region. The expression regulatory region may include, but is not limited to, a promoter, an operator sequence, a ribosome-binding site-encoding sequence, a sequence for regulating termination of transcription or translation, an enhancer, and the like. The replacement may be performed, specifically, by a method of replacing an endogenous promoter with a strong heterologous promoter, without being limited thereto.

Examples of the strong promoter known in the art may include CJ1 to CJ7 promoters (U.S. Patent No. US 7662943 B2), lac promoter, trp promoter, trc promoter, tac promoter, Lambda phage PR promoter, PL promoter, tet promoter, gapA promoter, SPL7 promoter, SPL13(sm3) promoter (U.S. Patent No. US 10584338 B2), O2 promoter (U.S. Patent No. US 10273491 B2), tkt promoter, and yccA promoter, without being limited thereto.

The modifying a nucleotide sequence encoding a start codon or a 5'-UTR region of a gene transcript encoding the polypeptide described in 3) above may be achieved, for example, by substituting the nucleotide sequence with a nucleotide sequence encoding another start codon having a higher expression level of the polypeptide than the endogenous start codon, without being limited thereto.

The modifying an amino acid sequence or a nucleotide sequence described in 4) and 5) above may be achieved by inducing mutation in the amino acid sequence of the polypeptide or the nucleotide sequence encoding the polypeptide by deletion, insertion, non-conservative, or conservative substitution, or any combination thereof, or by replacing the amino acid sequence or the nucleotide sequence with an amino acid sequence or a nucleotide sequence modified to have stronger activity or an amino acid sequence of a nucleotide sequence modified to increase the activity, in order to further enhance the activity of the polypeptide, without being limited thereto. The replacement may be performed, specifically, by inserting a polynucleotide into the chromosome by homologous recombination, without being limited thereto. In this regard, a vector used therefor may further include a selection marker to confirm insertion thereof into the chromosome. The selection marker will be described below.

The introducing a foreign polypeptide having the activity of the polypeptide described in 6) above may be achieved by introducing a foreign polynucleotide encoding a polypeptide exhibiting activity identical/similar to that of the polypeptide into a host cell. The origin or sequence of the foreign polynucleotide is not particularly limited as long as the foreign polynucleotide exhibits activity identical/similar to that of the polypeptide. A method used in the introduction may be appropriately selected by those of ordinary skill in the art. As the introduced polynucleotide is expressed in the host cell, the polypeptide is produced and the activity thereof may be increased.

The optimizing a codon of a polynucleotide encoding the polypeptide described in 7) above may be achieved by optimizing a codon to increase transcription or translation of an endogenous polynucleotide in a host cell, or by optimizing a codon to allow optimized transcription or translation of a foreign polynucleotide in a host cell.

The modifying or chemically modifying an exposed region selected by analyzing a three-dimensional structure of the polypeptide described in 8) above may be achieved, for example, by determining a template protein candidate according to similar between sequences based on comparison between information on a sequence of the polypeptide to be analyzed and a database that stores information on sequences of existing proteins, identifying the structure based thereon, selecting an exposed region to be modified or chemically modified, and modifying or chemically modifying the exposed region.

The enhancement of the activity of the regulator of acetate metabolism A as described above may be an increase in the activity or concentration (expression level) of the polypeptide compared with the activity or concentration of the regulator of acetate metabolism A expressed in the wild-type or non-modified microorganism strains, or an increase in an amount of a product obtained from the polypeptide, without being limited thereto.

More specifically, as used herein, the term "gene expression regulatory sequence", interchangeably used with "gene expression regulatory region", refers to a sequence operatively linked to a target gene so as to express the target gene and may include the modified polynucleotide of the present disclosure. As described above, the gene expression regulatory sequence of the present disclosure may refer to a promoter, an enhancer, or the like to perform transcription of a gene, and may be a concept further including an operator sequence to control transcription, a sequence encoding a suitable mRNA ribosome-binding site, and DNA regulating termination of transcription and translation.

In an embodiment of the present disclosure, the gene expression regulatory sequence may be a promoter, but is not limited thereto.

In an embodiment of the present disclosure, the expression of the regulator of acetate metabolism A may be enhanced by introducing a modification into the promotor or replacing the promoter with a promoter having stronger activity, but is not limited thereto.

As used herein, the term "promoter" refers to an untranslated nucleotide sequence including a binding site for a polymerase, located upstream a coding region, and having the activity of initiating transcription of a target gene into mRNA, *i.e.,* a region of DNA that leads to initiation of transcription of the gene when a polymerase binds thereto. The promoter may be located at the 5' region of an mRNA transcription initiation site.

As used herein, the term "operatively linked" refers to functionally linked to the sequence of a target gene such that a polynucleotide having the promoter activity of the present disclosure initiates and mediates transcription of the target gene. Operative linkage may be obtained using a genetic recombination technique known in the art and site-specific DNA cleavage and ligation may be performed using a restriction enzyme, a ligase, or the like, without being limited thereto.

In the present disclosure, the target gene refers to a gene encoding the target protein whose expression is to be controlled in a microorganism, specifically, the gene may be a gene encoding the regulator of acetate metabolism A, but is not limited thereto. More specifically, the gene may be *ramA* gene, but is not limited thereto.

In addition, the *ramA* gene may be an endogenous gene or a foreign gene and may include mutation to adjust activity. A sequence of the *ramA* gene may be easily obtained from a known database such as the GenBank of the National Institutes of Health (U.S.A.) by those skilled in the art.

Another aspect of the present disclosure provides an L-branched-chain amino acid-producing microorganism including a polynucleotide having promoter activity and including substitution of a nucleotide with a different nucleotide at one or more corresponding positions selected from positions 34, 36, 37, 41, and 43 of a nucleotide sequence as set forth in SEQ ID NO: 1.

As used herein, the term "polynucleotide" refers to a DNA strand having a certain minimum length as a polymer of nucleotides in which nucleotide monomers are linked to each other in the form of a long chain by covalent bonds.

In consideration of the descriptions of the "promoter", the term "polynucleotide having promoter activity" may be used interchangeably with "modified polynucleotide", "modified promoter", or "modified ramA promoter" in the present disclosure, and all of the above-described terms may be used herein.

In this regard, the term "modification" refers to a genetically or non-genetically stable phenotypic change and may be used interchangeably with "mutation" herein.

Specifically, the modified polynucleotide of the present disclosure may have a modified (increased) promoter activity compared to a polynucleotide not having modification. Thus, expression of the *ramA* gene that is a target gene operatively linked to the modified polynucleotide of the present disclosure and activity of the protein encoded by the *ramA* gene may be adjusted (increased), and also expression of other genes as well as the target gene may be adjusted.

In view of the objects of the present disclosure, the polynucleotide having promoter activity refers to a polynucleotide capable of expressing a protein involved in an increase in production of amino acids, specifically, branched-chain amino acids, more specifically, amino acids including leucine, valine, and isoleucine, but is not limited thereto.

As used herein, the term "polynucleotide sequence as set forth in SEQ ID NO: 1" may refer to a promoter sequence of a gene encoding the regulator of acetate metabolism A (RamA).

The polynucleotide having promoter activity of the present disclosure is a modified polynucleotide not having a naturally derived sequence but having promoter activity, and expression of the target protein operatively linked thereto may be increased compared to the polynucleotide sequence as set forth in SEQ ID NO: 1.

Specifically, the modified polynucleotide of the present disclosure may be a polynucleotide in which the nucleotide sequence as set forth in SEQ ID NO: 1, *i.e.,* the promoter sequence of the *ramA* gene is modified, and at least one nucleotide selected from the 34^{th}, 36^{th}, 37^{th}, 41^{st}, and 43^{rd} nucleotides of the sequence may be substituted with a different nucleotide.

More specifically, the modified polynucleotide of the present disclosure may include substitution with T at the 34^{th} nucleotide; substitution with T at the 36^{th} nucleotide; substitution with G at the 37^{th} nucleotide; substitution with T at the 41^{st} nucleotide; substitution with A at the 43^{rd} nucleotide; or any combination thereof in the nucleotide sequence as set forth in SEQ ID NO: 1, without being limited thereto. By the modification, the polynucleotide of the present disclosure may consist of one nucleotide sequence selected from SEQ ID NOS: 3 to 5.

In an embodiment, the modified polynucleotide of the present disclosure may include substitution with T at the 34^{th} nucleotide; substitution with T at the 36^{th} nucleotide; and substitution with G at the 37^{th} nucleotide in the nucleotide sequence as set forth in SEQ ID NO: 1. In this case, the modified polynucleotide of the present disclosure may consist of SEQ ID NO: 5.

In another embodiment, the modified polynucleotide of the present disclosure may include substitution with T at the 41^{st} nucleotide; and substitution with A at the 43^{rd} nucleotide in the nucleotide sequence as set forth in SEQ ID NO: 1. In this case, the modified polynucleotide of the present disclosure may consist of SEQ ID NO: 4.

In another embodiment, the modified polynucleotide of the present disclosure may include substitution with T at the 34^{th} nucleotide; substitution with T at the 36^{th} nucleotide; substitution with G at the 37^{th} nucleotide; substitution with T at the 41^{st} nucleotide; and substitution with A at the 43^{rd} nucleotide in the nucleotide sequence as set forth in SEQ ID NO: 1. In this case, the modified polynucleotide of the present disclosure may consist of SEQ ID NO: 3.

The L-branched-chain amino acid-producing microorganism of the present disclosure may be a microorganism including a polynucleotide which has promoter activity and includes substitution with T at the 34^{th} nucleotide; substitution with T at the 36^{th} nucleotide; substitution with G at the 37^{th} nucleotide; substitution with T at the 41^{st} nucleotide; substitution with A at the 43^{rd} nucleotide; or any combination thereof in the nucleotide sequence as set forth in SEQ ID NO: 1.

Specifically, the L-branched-chain amino acid-producing microorganism of the present disclosure may be a microorganism including a polynucleotide having promoter activity and consisting of one nucleotide sequence selected from SEQ ID NOS: 3 to 5 or a nucleotide sequence having at least 80% or more and less than 100% of sequence homology therewith. The modified polynucleotide of the present disclosure will be described below in more detail.

As used herein, the term "branched-chain amino acid" refers to an amino acid having a branched alkyl group at a side chain and includes valine, leucine, and isoleucine. Specifically, in the present disclosure, the branched-chain amino acid may be an L-branched-chain amino acid, and the L-branched-chain amino acid may be L-valine, L-leucine, and L-isoleucine, without being limited thereto.

As used herein, the term "branched-chain amino acid-producing microorganism" includes all of wild-type microorganisms and microorganisms in which natural or artificial genetic modification occurs and may be a microorganism including genetic modification to produce a target branched-chain amino acid or having improved activity in which a particular mechanism is weakened or enhanced via introduction of a foreign gene or enhancement or inactivation of activity of an endogenous gene. In the present disclosure, "microorganism capable of producing an L-branched-chain amino acid" may be used interchangeably with "branched-chain amino acid-producing microorganism" and "microorganism having the branched-chain amino acid producing ability".

In view of the objects of the present disclosure, the microorganism may be any microorganism capable of producing a branched-chain amino acid and including the modified polynucleotide of the present disclosure. Specifically, the branched-chain amino acid-producing microorganism may be a microorganism characterized in that the ability to produce a target branched-chain amino acid is increased by including the modified polynucleotide. Specifically, in the present disclosure, the branched-chain amino acid-producing microorganism or the microorganism having the ability to produce a branched-chain amino acid may be a microorganism in which some of the genes in a biosynthesis pathway of the branched-chain amino acid are enhanced or weakened or a microorganism in which some of the genes in the decomposition pathway of the branched-chain amino acid are enhanced or weakened, without being limited thereto.

In an embodiment, in the present disclosure, the microorganism of the genus *Corynebacterium* having the branched-chain amino acid producing ability may refer to a microorganism of the genus *Corynebacterium* including the modified polynucleotide of the present disclosure or transformed with a vector including a gene encoding the polynucleotide of the present disclosure to have improved branched-chain amino acid producing ability. The "microorganism of the genus *Corynebacterium* having enhanced branched-chain amino acid producing ability" refers to a microorganism having enhanced ability to produce branched-chain amino acid compared to the ability of the amino acid possessed by a parent strain before transformation or a non-modified microorganism. The "non-modified microorganism" does not exclude strains having mutation that may naturally occur in microorganisms and refers to a microorganism not including the polynucleotide of the present disclosure or a microorganism not transferred with a vector including the polynucleotide of the present disclosure.

The microorganism of the genus *Corynebacterium* may specifically include *Corynebacterium glutamicum, Corynebacterium ammoniagenes, Brevibacterium lactofermentum, Brevibacterium flavum, Corynebacterium thermoaminogenes, Corynebacterium efficiens, Corynebacterium stationis,* and the like, without being limited thereto.

As used herein, the term "vector" refers to an artificial DNA molecule including a genetic material to express a target polypeptide in a proper host cell, specifically, a DNA construct including a nucleotide sequence of a polynucleotide encoding the target polypeptide and operatively linked to a suitable expression regulatory region capable of expressing a target gene. After a suitable host cell is transformed with the vector, the vector may replicate or function independently from the host genome or may be integrate into the genome.

The vector used in the present disclosure is not particularly limited and any vector known in the art may be used. Examples of conventional vectors may include a natural or recombinant plasmid, cosmid, virus, and bacteriophage. For example, as the phage vector or the cosmid vector, pWE15, M13, MBL3, MBL4, IXII, ASHII, APII, t10, t11, Charon4A, Charon21A, and the like may be used. As the plasmid vector, pDZ-based, pBR-based, pUC-based, pBluescriptll-based, pGEM-based, pTZ-based, pCL-based, pET-based, and the like may be used. Specifically, pDZ, pDC, pDCM2, pACYC177, pACYC184, pCL, pECCG117, pUC19, pBR322, pMW118, and pCC1BAC vectors may be used.

For example, a target polynucleotide may be inserted into the chromosome by using a vector for chromosomal insertion. The insertion of the polynucleotide into the chromosome may be performed by any method known in the art, e.g., homologous recombination, but is not limited thereto. The polynucleotide may further include a selection marker to confirm chromosomal insertion. The selection marker is used to select cells transformed with the vector, *i.e.,* to identify whether a target nucleic acid molecule is inserted or not, and markers providing selective phenotypes such as drug tolerance, nutrient requirement, resistance to cytotoxic agents, or expression of surface polypeptide may be used. Since only cells expressing the selection marker are able to survive or show different phenotypes under the environment treated with a selective agent, transformed cells may be selected.

As used herein, the term "transformation" refers to a process of introducing a vector including a target polynucleotide into a host cell or microorganism in such a way that the polynucleotide is expressed in the host cell. The transformed polynucleotide may be either in a form inserted into the chromosome of the host cell or in a form located outside the chromosome as long as the polypeptide is expressed in the host cell. In addition, the polynucleotide may include DNA and/or RNA encoding the target polypeptide. The polynucleotide may be introduced into the host cell in any form as long as the polynucleotide is introduced into the host cell and expressed therein. For example, the polynucleotide may be introduced into the host cell in the form of an expression cassette that is a gene construct including all of the essential elements required for self-replication. The expression cassette may generally include a promoter operatively linked to the polynucleotide, a transcription termination signal, a ribosome binding site, and a translation termination signal. The expression cassette may be in the form of a self-replicable expression vector. Also, the polynucleotide may be introduced into the host cell in its original form and operatively linked to a sequence required for the expression in the host cell, without being limited thereto.

Another aspect of the present disclosure provides a method for producing an L-branched-chain amino acid including culturing the microorganism in a culture medium.

In addition, the method for producing the L-branched-chain amino acid may further include recovering or separating a target substance from the culture medium or the microorganism.

The "polynucleotide", microorganism of the genus *Corynebacterium",* "vector" and "L-branched-chain amino acid" are as described above.

As used herein, the term "culturing" refers to growing the microorganism of the present disclosure in an appropriately adjusted environment. A culturing process of the present disclosure may be performed using appropriate culture media and culture conditions well known in the art. The culturing process may be appropriately adjusted by those skilled in the art in accordance with a selected strain. Specifically, the culturing may be performed by a batch culture method, a continuous culture method, and a fed-batch culture method, without being limited thereto.

As used herein, the term "culture medium" refers to a material in which nutrients required for culturing the microorganism of the present disclosure are mixed as main elements and supplies nutrients and growth factors as well as water which are essential for survival and growth. Specifically, although culture media and other culturing conditions for the microorganism of the genus *Corynebacterium* of the present disclosure are not particularly limited as long as the media are commonly used in culturing microorganisms, the microorganism of the present disclosure may be cultured in an ordinary medium containing appropriate carbon sources, nitrogen sources, phosphorus sources, inorganic compounds, amino acids, and/or vitamins under aerobic conditions while adjusting temperature, pH, and the like.

Specifically, the culture medium for the microorganism of the genus *Corynebacterium* is disclosed in a document ("Manual of Methods for General Bacteriology" by the American Society for Bacteriology (Washington D.C., U.S.A., 1981)).

In the present disclosure, as the carbon sources, carbohydrates such as glucose, sucrose, lactose, fructose, and maltose; sugar alcohols such as mannitol and sorbitol; organic acids such as pyruvic acid, lactic acid, and citric acid; and amino acids such as glutamic acid, methionine, and lysine may be used. In addition, natural organic nutrients such as starch hydrolysates, molasses, blackstrap molasses, rice bran, cassava, sugar cane bagasse, and corn steep liquor may be used, and specifically, carbohydrates such as glucose and sterile pretreated molasses (i.e., molasses converted into reduced sugars) may be used, and suitable amounts of any other carbon sources may also be used without limitation. These carbon sources may be used alone or in combination of at least two thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids, e.g., glutamic acid, methionine, and glutamine, peptone, NZ-amine, meat extract, yeast extract, malt extract, corn steep liquor, casein hydrolysate, fish or degradation products thereof, and defatted soybean cake or degradation products thereof may be used. These nitrogen sources may be used alone or in combination of at least two thereof, without being limited thereto.

As the phosphorus sources, monopotassium phosphate, dipotassium phosphate, or sodium-containing salts corresponding thereto may be used. As inorganic compounds, sodium chloride, calcium chloride, iron chloride, magnesium sulfate, iron sulfate, manganese sulfate, calcium carbonate, and the like may be used. Also, amino acids, vitamins, and/or appropriate precursors may further be included. These components or precursors may be added to the culture medium in a batch or continuous process, without being limited thereto.

In addition, during the process of culturing the microorganism of the present disclosure, a pH of the culture medium may be adjusted by adding a compound such as ammonium hydroxide, potassium hydroxide, ammonia, phosphoric acid, and sulfuric acid in an appropriate method. In addition, during the culturing process, foam formation may be prevented by using a defoaming agent such as fatty acid polyglycol ester. Also, oxygen or oxygen-containing gas may be injected into the culture medium to maintain the culture medium in aerobic conditions, or nitrogen, hydrogen, or carbon dioxide gases may be injected into the culture medium to maintain the culture medium in anaerobic and microaerobic conditions without injecting any other gases therefor, without being limited thereto.

In the present disclosure, the culturing temperature may be maintained at 20°C to 45°C, specifically 25°C to 40°C, and the culturing may be performed for about 10 to 160 hours, without being limited thereto.

The L-branched-chain amino acid produced by the culturing of the present disclosure may be released into the culture medium or remain in the cells.

The method for producing an L-branched-chain amino acid according to the present disclosure may further include preparing the microorganism of the present disclosure, preparing a culture medium for culturing the strain, or any combination thereof (regardless of the order, in any order), for example, before the culturing.

The method for producing an L-branched-chain amino acid according to the present disclosure may further include recovering the L-branched-chain amino acid from the culture medium (in which the culturing has been performed) or the microorganism of the present disclosure. The recovering step may be additionally performed after the culturing process.

The recovering step may be performed by collecting the target L-branched-chain amino acid using an appropriate method known in the art according to methods of culturing the microorganism according to the present disclosure, such as a batch, continuous, or fed-batch method. For example, centrifugation, filtration, treatment with a protein precipitating agent (salting out), extraction, ultrasonic disintegration, ultrafiltration, dialysis, various chromatographic methods such as molecular sieve chromatography (gel permeation), adsorption chromatography, ion exchange chromatography, and affinity chromatography, HPLC, SMB, and any combination thereof may be used. The target L-branched-chain amino acid may be recovered from the culture medium or the microorganism using any appropriate method known in the art.

In addition, the method for producing an L-branched-chain amino acid according to the present disclosure may further include a purifying step. The purification may be performed using an appropriate method known in the art. As an example, when the method for producing an L-branched-chain amino acid according to the present disclosure includes both the recovering step and the purifying step, the recovering step and the purifying step may be performed continuously or discontinuously regardless of the order or may be performed simultaneously or as one integrated step, without being limited thereto.

Another aspect of the present disclosure provides a polynucleotide having promoter activity and including substitution of at least one nucleotide selected from the 34^{th}, 36^{th}, 37^{th}, 41 ^{st}, and 43^{rd} nucleotides of the nucleotide sequence as set forth in SEQ ID NO: 1 with a different nucleotide.

The "polynucleotide" and "promoter" are as described above.

Also, the sequence of the modified polynucleotide of the present disclosure may be modified by mutagenesis well known in the art, for example, direct evolution and site-directed mutagenesis.

Thus, the modified polynucleotide of the present disclosure may include a polynucleotide having a nucleotide sequence in which the 34^{th} base is fixed as T; the 36^{th} base is fixed as T; the 37^{th} base is fixed as G; the 41^{st} base is fixed as T; and the 43^{rd} base is fixed as A, and the other part of the nucleotide sequence has at least 70%, 80%, 85%, 90%, 95%, 96%, 97%, 98%, or 99% homology or identity with the nucleotide sequence of SEQ ID NO: 3.

Also, the modified polynucleotide of the present disclosure may include a polynucleotide having a nucleotide sequence in which the 41^{st} base is fixed as T; and the 43^{rd} base is fixed as A, and the other part of the nucleotide sequence has at least 70%, 80%, 85%, 90%, 95%, 96%, 97%, 98%, or 99% homology or identity with the nucleotide sequence of SEQ ID NO: 4.

Also, the modified polynucleotide of the present disclosure may include a polynucleotide having a nucleotide sequence in which in which the 34^{th} base is fixed as T; the 36^{th} base is fixed as T; and the 37^{th} base is fixed as G, and the other part of the sequence has at least 70%, 80%, 85%, 90%, 95%, 96%, 97%, 98%, or 99% homology or identity with the nucleotide sequence of SEQ ID NO: 5.

In this case, the nucleotide sequence having the homology or identity may exclude a sequence having 100% identity or may be a sequence having an identity less than 100%.

It is obvious that any polynucleotide having a nucleotide sequence including deletion, modification, substitution, or addition of one or several nucleotides other than the 34^{th}, 36^{th}, 37^{th}, 41^{st}, or 43^{rd} position is within the scope of the present disclosure as long as the nucleotide sequence retains homology and biological activity identical or equivalent to those of at least one nucleotide sequence selected from SEQ ID NOS: 3 to 5.

As used herein, the term "homology" or "identity" refers to the degree of relatedness between two given amino acid sequences or nucleotide sequences and may be expressed as a percentage. The terms homology and identity may often be used interchangeably.

Sequence homology or identity of conserved polynucleotides or polypeptides may be determined by standard alignment algorithm and default gap penalties established by a program may be used together therewith. Substantially, homologous or identical sequences may generally hybridize with each other in whole or in part under moderate or highly stringent conditions. It is obvious that hybridization includes hybridization of a polynucleotide with a polynucleotide including a general codon or a codon in consideration of codon degeneracy.

Homology, similarity, or identity between two sequences of polynucleotides or polypeptides may be determined using any computer algorithm known in the art, e.g., "FASTA" program, using default parameters introduced by Pearson et al. (1988) Proc. Natl. Acad. Sci. USA 85:2444. Alternatively, the homology, similarity, or identity may be determined using the Needleman-Wunsch algorithm (Needleman and Wunsch, 1970, J. Mol. Biol. 48:443-453) as implemented in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST, from the National Center for Biotechnology Information database, or ClustalW.

The homology, similarity, or identity between polynucleotides or polypeptides may be determined by comparing sequence information using a GAP computer program as introduced by Needleman et al., (1970), J Mol Biol. 48:443 as disclosed by Smith and Waterman, Adv. Appl. Math (1981) 2:482. Briefly, the GAP program defines similarity as the number of aligned symbols (i.e., nucleotides or amino acids) which are similar, divided by the total number of symbols in a shorter of two sequences. Default parameters for the GAP program may include: (1) a binary comparison matrix (containing a value of 1 for identities and 0 for non identifies) and the weighted comparison matrix of Gribskov, et al. (1986), Nucl. Acids Res. 14:6745 as described by Schwartz and Dayhoff, eds., Atlas Of Protein Sequence and Structure, National Biomedical Research Foundation, pp 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

In addition, the modified polynucleotide of the present disclosure may include various modifications made in a coding region provided not to change the nucleotide sequence by codon degeneracy or in consideration of codons preferred by a living organism in which the polynucleotide is expressed. Also, the polynucleotide may include any nucleotide sequence having promoter activity and hybridized with a probe constructed using known gene sequence, e.g., a nucleotide sequence entirely or partially complementary to the nucleotide sequence under stringent conditions, to include at least one substituted nucleotide in the nucleotide sequence of SEQ ID NO: 1, without limitation. The term "stringent conditions" refers to conditions which permit specific hybridization between polynucleotides. Such conditions are disclosed in detail in known documents (e.g., J. Sambrook *et al.).* For example, the conditions may include performing hybridization between genes having a high homology, e.g., a homology of 40% or more, specifically 70% or more, 80% or more, 85% or more, 90% or more, more specifically 95% or more, even more specifically 97% or more, and most specifically 99% or more, without performing hybridization between genes having a homology or identity lower than those described above, or performing hybridization once, specifically two or three times, under conventional washing conditions for Southern hybridization at a salt concentration and temperature of 60°C, 1×SSC, and 0.1% SDS, specifically 60°C, 0.1×SSC, 0.1% SDS, and more specifically 68°C, 0.1 xSSC, and 0.1% SDS.

Hybridization requires that two nucleic acids have complementary sequences, although bases mismatch according to the degree of stringency of hybridization. The term "complementary" is used to describe the relationship between bases of nucleotides capable of hybridizing with each other. For example, with respect to DNA, adenosine is complementary to thymine, and cytosine is complementary to guanine. Thus, the present disclosure may include not only a substantially similar nucleic acid sequence but also a nucleic acid fragment isolated but complementary to the entire sequence.

Specifically, the polynucleotides having homology or identity may be detected using the above-described hybridization conditions including a hybridization process at a Tm value of 55°C. Also, the Tm value may be, but is not limited to, 60°C, 63°C, or 65°C, and may be appropriately adjusted by those skilled in the art according to the intended purposes.

An appropriate degree of stringency for hybridization of polynucleotides may depend on lengths of the polynucleotides and a degree of complementarity and parameters thereof are well known in the art (Sambrook *et al., supra,* 9.50-9.51, 11.7-11.8).

In particular, the expression "the modified polynucleotide consists of one nucleotide sequence selected from SEQ ID NOS: 3 to 5 or a nucleotide sequence having at least 80% or more and less than 100% of sequence homology" does not exclude addition and/or deletion and/or mutation of a nucleotide which may occur during a ligation process to a target gene, e*.g.,* using a restriction enzyme, in the case where the polynucleotide is used in a state of being ligated to the target gene, as a promoter.

For example, the polynucleotide consisting of one nucleotide sequence selected from SEQ ID NOS: 3 to 5 and having promoter activity may also include a polynucleotide hybridized to a nucleotide sequence entirely or partially complementary to one nucleotide sequence selected from SEQ ID NOS: 3 to 5 under stringent conditions to have the promoter activity of the present disclosure.

The microorganism including the modified polynucleotide of the present disclosure is characterized in that production of branched-chain amino acids including valine, leucine, or isoleucine increases. While wild-type strains belonging to the genus *Corynebacterium* cannot produce or produce trace amounts of branched-chain amino acids, the polynucleotide having the promoter activity of the present disclosure is significant in that production of branched-chain amino acids is increased thereby.

### [Mode for Invention]

Hereinafter, the present disclosure will be described in more detail with reference to the following examples. However, the following examples are merely presented to exemplify the present disclosure, and the scope of the present disclosure is not limited thereto.

### Example 1. Selection of Mutant Strains Having Enhanced Valine Producing Ability via Artificial Mutagenesis

### Example 1-1. Induction of Artificial Mutation by UV Radiation

In order to select a mutant strain having enhanced ability to produce valine that is a representative branched-chain amino acid, *Corynebacterium glutamicum* KCCM11201P (Korean Patent No. 10-1117022) as a valine-producing strain was smeared on an agar-containing nutrient medium and cultured at 30°C for 36 hours. Several hundreds of colonies were obtained therefrom and exposed to UV rays at room temperature to induce random mutation on genomes of the strains.

### Example 1-2. Fermentation Titer Evaluation and Selection of Mutation-induced Strain

In order to select a mutant strain having enhanced L-valine producing ability compared to *Corynebacterium glutamicum* KCCM11201P used as a parent strain, a fermentation titer test was performed on the strains in which random mutation was induced. After subculturing each of the colonies in a nutrient medium, each of the strains was inoculated onto a 250 mL corner-baffled flask containing 25 mL of a production medium and cultured while shaking at 30°C for 72 hours at 200 rpm. Then, concentrations of L-valine were analyzed using HPLC, and the analyzed concentrations of L-valine are shown in Table 1 below.

### Nutrient medium (pH 7.2)

10 g of glucose, 5 g of meat gravy, 10 g of polypeptone, 2.5 g of sodium chloride, 5 g of yeast extract, 20 g of agar, 2 g of urea (based on 1 L of distilled water)

### Production Medium (pH 7.0)

100 g of glucose, 40 g of ammonium sulfate, 2.5 g of soy protein, 5 g of corn steep solids, 3 g of urea, 1 g of dibasic potassium phosphate, 0.5 g of magnesium sulfate heptahydrate, 100 µg of biotin, 1 mg of thiamine-HCI, 2 mg of calcium pantothenate, 3 mg of nicotinamide, and 30 g of calcium carbonate (based on 1 L of distilled water)

**Table 1**

| | Strain name | L-Valine (g/L) |
|---|---|---|
| Control | KCCM11201P | 2.8 |
| Experimental group | A1 | 2.9 |
| | A2 | 2.5 |
| | A3 | 3.5 |
| | A4 | 3.0 |
| | A5 | 1.5 |
| | A6 | 1.2 |
| | A7 | 4.2 |
| | A8 | 3.9 |
| | A9 | 2.8 |
| | A10 | 2.4 |
| | A11 | 3.1 |
| | A12 | 3.3 |
| | A13 | 3.8 |
| | A14 | 2.7 |
| | A15 | 2.9 |

In comparison with the KCCM11201P strain used as a control, A7 strain, whose production of valine was increased the most, was selected (see Table 1).

### Example 2. Confirmation of Mutation By Gene Sequencing

Major genes of the A7 strain having enhanced valine producing ability were sequenced and compared with those of the KCCM11201P strain and wild-type *Corynebacterium glutamicum* ATCC14067 strain. As a result, it was confirmed that the A7 strain contains mutation at the promoter position of regulator of acetate metabolism A.

Specifically, it was confirmed that the A7 strain had a nucleotide sequence of SEQ ID NO: 2 including mutation at the promoter region (SEQ ID NO: 1) of the *ramA* gene.

In the following examples, effects of modification inserted into a specific position of the promoter region of the *ramA* gene and effects of enhanced expression of RamA by improvement or substitution of the promoter of the *ramA* gene on production of valine, isoleucine, and leucine, which are branched-chain amino acids of a microorganism of the genus *Corynebacterium,* were examined.

### Example 3. Construction of Strain Introduced with Modification and Confirmation of Valine Producing Ability

### Example 3-1. Introduction of Promoter Modification into Corynebacterium glutamicum KCCM11201P Strain and Evaluation of L-Valine Producing Ability

In order to insert a *ramA* gene promoter-modified polynucleotide represented by SEQ ID NO: 2 into *Corynebacterium glutamicum* KCCM11201P, a vector including a target modification was prepared. Specifically, genomic DNA of the A7 strain was extracted using a G-spin Total DNA Extraction Mini Kit (Intron, Cat. No 17045) in accordance with protocols of the kit, and PCR was performed using the genomic DNA as a template. The PCR was performed under the following conditions: denaturation at 94°C for 5 minutes; 25 cycles of denaturation at 94°C for 30 seconds, annealing at 55°C for 30 seconds, and polymerization at 72°C for 150 seconds; and then polymerization at 72°C for 7 minutes, and a PCR product (hereinafter, referred to as "modification-introduced fragment 1") of 1114 bp was obtained using SEQ ID NOS: 9 and 10.

After treating the obtained modification-introduced fragment 1 with the restriction enzyme Xbal (New England Biolabs, Beverly, MA), the modification-introduced fragment 1 was ligated to a pDZ vector (Korean Patent No. 10-0924065 and International Patent Application Publication No. 2008-033001) treated with the same restriction enzyme using a T4 ligase (New England Biolabs, Beverly, MA). After transforming E. *coli* DH5α with the constructed gene, transformed strains were selected in an LB medium containing kanamycin and DNA was obtained therefrom using a DNA-spin plasmid DNA purification kit (iNtRON) to prepare a pDZ-Pm-ramA vector including the modification-introduced fragment 1.

**Table 2**

| Primer | Base sequence | SEQ ID NO: |
|---|---|---|
| Pm(TATAAT)-F1 | gctctagaTAGGCCGGTTCGGACTCGCCCTG CC | SEQ ID NO: 9 |
| Pm (TATAAT)-R1 | gc**tctaga**aacgtgcgcgcagtcatggtgactt | SEQ ID NO: 10 |

*Corynebacterium glutamicum* KCCM11201P was transformed with the pDZ-Pm-ramA vector via chromosomal homologous recombination (van der Rest et al., Appl Microbiol Biotechnol 52:541-545, 1999). Strains whose chromosome was inserted with the vector by homologous sequence recombination were selected in a culture medium containing kanamycin (25 mg/ℓ). Then, PCR was performed on the *Corynebacterium glutamicum* transformants in which secondary recombination was completed using SEQ ID NOS: 9 and 10 and strains in which modification was inserted into the promoter at an upstream region of *ramA* (SEQ ID NO: 1) on the chromosome were confirmed. The recombinant strain was named *Corynebacterium glutamicum* KCCM11201P-Pm-ramA.

For comparison of valine producing ability between valine-producing *Corynebacterium glutamicum* KCCM11201P and KCCM11201P-Pm-ramA, flask evaluation was performed. After subculturing each of the strains in a nutrient medium, each of the strains was inoculated onto a 250 mL corner-baffled flask containing 25 mL of a production medium, and cultured while shaking at 30°C for 72 hours at 200 rpm. Then, concentrations of L-valine were analyzed using HPLC, and the analyzed concentrations of L-valine are shown in Table 3 below.

### Nutrient Medium (pH 7.2)

10 g of glucose, 5 g of meat gravy, 10 g of polypeptone, 2.5 g of sodium chloride, 5 g of yeast extract, 20 g of agar, 2 g of urea (based on 1 L of distilled water)

### Production Medium (pH 7.0)

100 g of glucose, 40 g of ammonium sulfate, 2.5 g of soy protein, 5 g of corn steep solids, 3 g of urea, 1 g of dibasic potassium phosphate, 0.5 g of magnesium sulfate heptahydrate, 100 µg of biotin, 1 mg of thiamine-HCI, 2 mg of calcium pantothenate, 3 mg of nicotinamide, 30 g of calcium carbonate (based on 1 L of distilled water)

**Table 3**

| L-valine producing ability of KCCM11201 P and KCCM11201 P-Pm-ramA | | | | |
|---|---|---|---|---|
| Strain | L-valine (g/L) | | | |
| | Batch 1 | Batch 2 | Batch 3 | Average |
| KCCM11201P | 2.6 | 2.5 | 2.7 | 2.6 |
| KCCM11201 P-Pm-ramA | 3.2 | 3.3 | 3.1 | 3.2 |

As a result, it was confirmed that the L-valine producing ability of the KCCM11201P-Pm-ramA strain was enhanced by about 23% compared to that of KCCM11201P.

### Example 3-2. Construction of Mutant Strain of Corynebacterium Glutamicum KCCM11201P in which Promoter Is Improved and Substituted and Evaluation of L-Valine Producing Ability of Constructed Strain

As shown in the results of Example 3-1 above, it was confirmed that the valine producing ability was enhanced by modifying the *ramA* gene promoter, and thus vectors for improving or substituting the *ramA* promoter were constructed based on the modified promoter of SEQ ID NO: 2 to further increase expression of *ramA.*

In order to construct vectors including modification, primer 3 (SEQ ID NO: 11) to primer 10 (SEQ ID NO: 18) of Table 4 were synthesized to have an xbal restriction enzyme region at the 5' terminal and the 3' terminal.

The improved *ramA* promoters were named Pm1, Pm2, and Pm3-ramA, and a primer pair of SEQ ID NOS: 11 and 13; and a primer pair of SEQ ID NOS: 12 and 14 were used to construct Pm1-ramA and a primer pair of SEQ ID NOS: 11 and 15 and a primer pair of SEQ ID NOS: 12 and 14 were used to construct Pm2-ramA. Also, a primer pair of SEQ ID NOS: 11 and 17; and a primer pair of SEQ ID NOS: 12 and 18 was used to construct Pm3-ramA .

PCR was performed using each of the primers and chromosomal DNA of wild-type *Corynebacterium glutamicum* as a template [Sambrook *et al.,* Molecular Cloning, a Laboratory Manual (1989), Cold Spring Harbor Laboratories].

In this case, the PCR was performed under the following conditions: denaturation at 95°C for 5 minutes; 30 cycles of denaturation at 94°C for 30 seconds, annealing at 56°C for 30 seconds, and polymerization at 72°C for 1 minute; and then polymerization at 72°C for 7 minutes.

Then, a PCR product obtained from the above-described process and the previously prepared pDZ-Pm-ramA vector were treated with the xbal restriction enzyme, followed by fusion cloning. The fusion cloning was performed using an InFusion^{®} HD Cloning Kit (Clontech). *E. coli* DH5α was transformed therewith and smeared on an LB solid medium containing kanamycin (25 mg/f). Colonies transformed with plasmids into which a target gene was inserted were selected by PCR and the plasmids was obtained by extraction and named pDZ-Pm1-ramA, pDZ-Pm2-ramA, and pDZ-Pm3-ramA, respectively.

**Table 4**

| Primer | Base sequence | SEQ ID NO: |
|---|---|---|
| Primer 3 | gctcggtacccggggatcctctagataggccggttcggactcgccc tgcc | SEQ ID NO: 11 |
| Primer 4 | ttacgccaagcttgcatgctctagaaacgtgcgcgcagtcatggtg actt | SEQ ID NO: 12 |
| Primer 5 | CGA CAA GGG TCC ATT ATA CCA CAC CTT TGG GGG T | SEQ ID NO: 13 |
| Primer 6 | acccccaaaggTgTGgtaTaAtgGacccttgtcg | SEQ ID NO: 14 |
| primer 7 | TCG ACA AGG GTA CAT TAT ACT TCC CCT TT | SEQ ID NO: 15 |
| Primer 8 | aaaggggaagtaTaAtgtacccttgtcga | SEQ ID NO: 16 |
| Primer 9 | AAG GGT ACA GTG TAC CAC ACC TTT GGG GGT I | SEQ ID NO: 17 |
| Primer 10 | acccccaaaggTgTGgtacactgtaccctt | SEQ ID NO: 18 |
| Primer 11 | attcgagctcggtacccggtctagatcaagaaactgcaggtgtgta ccga | SEQ ID NO: 19 |
| Primer 12 | CAT CGG TAG GCT ATG CCG GCG GTA CCT TCA GAT TTC CTC CTG CTT TAC AC | SEQ ID NO: 20 |
| Primer 13 | gtaccgccggcatagcctaccgatg | SEQ ID NO: 21 |
| Primer 14 | AGT GTT TCC TTT CGT TGG GTA CGT A | SEQ ID NO: 22 |
| Primer 15 | tacgtacccaacgaaaggaaacactgtggatacccagcggatta aagatg | SEQ ID NO: 23 |
| Primer 16 | TGC ATG CCT GCA GGT CGA CTC TAG AAT CGC GGC GCA GAT CCT CAT CGG TC | SEQ ID NO: 24 |

Also, separately, in order to substitute the ramA promoter with Pcj7 that is a stronger promoter, primer 11 (SEQ ID NO: 19) to primer 16 (SEQ ID NO: 24) of Table 4 were synthesized to have an xbal restriction enzyme region at the 5' terminal and the 3'terminal.

A pDZ-Pcj7-ramA vector was constructed in the same manner as the method of constructing vectors in Example 3-1 described above using a primer pair of SEQ ID NOS: 19 and 20; a primer pair of SEQ ID NOS: 21 and 22; and a primer pair of SEQ ID NOS: 23 and 24.

*Corynebacterium glutamicum* KCCM11201P was transformed with the pDZ-Pm1-ramA, pDZ-Pm2-ramA, pDZ-Pm3-ramA, and pDZ-Pcj7-ramA vectors by chromosomal homologous recombination (van der Rest et al., Appl Microbiol Biotechnol 52:541-545, 1999). Strains whose chromosome was inserted with the vector by homologous sequence recombination were selected in a culture medium containing kanamycin (25 mg/f). Then, PCR was performed using the *Corynebacterium glutamicum* transformants in which secondary recombination was completed using SEQ ID NOS: 9 and 10 and strains in which the *ramA* promoter was improved and substituted with the Pcj7 promoter were confirmed.

Among the recombinant strains, *Corynebacterium glutamicum* KCCM11201P-Pm1-ramA, KCCM11201P-Pm2-ramA, and KCCM11201P-Pm3-ramA were named CA08-1518, CA08-1519, and CA08-1520, respectively, and deposited with the Korean Culture Center of Microorganisms (KCCM), recognized as an international depositary authority under the Budapest Treaty, on April 27, 2020, under the Accession Numbers of KCCM12704P, KCCM12705P, and KCCM12706P, respectively.

Also, the strain substituted with the Pcj7 promoter was named KCCM11201P-Pcj7-ramA. Subsequently, valine producing ability was evaluated in the same manner as in Example 3-1 above and the results are shown in Table 5 below.

**Table 5**

| Strain | L-valine (g/L) | | | |
|---|---|---|---|---|
| | Batch 1 | Batch 2 | Batch 3 | Average |
| KCCM11201P | 2.6 | 2.5 | 2.7 | 2.6 |
| KCCM11201P-Pm1-ramA(CA08-1518) | 3.2 | 3.4 | 3.3 | 3.3 |
| KCCM11201P-Pm2-ramA(CA08-1519) | 3.1 | 3.2 | 3.3 | 3.2 |
| KCCM11201P-Pm3-ramA(CA08-1520) | 3.2 | 3.0 | 3.1 | 3.1 |
| KCCM11201P-Pcj7-ramA | 2.9 | 3.1 | 3.0 | 3.0 |

Based on the results of Table 5, it was confirmed that the KCCM11201 P-Pm1-ramA (CA08-1518), KCCM11201P-Pm2-ramA (CA08-1519) and KCCM11201P-Pm3-ramA (CA08-1520) strains including the improved promoter compared to the KCCM11201P strain had L-valine production increased by about 27%, 23%, and 19%, respectively, which are similar to or higher than L-valine producing ability of the KCCM11201P-Pcj7-ramA strain substituted with the stronger promoter.

### Example 3-3: Construction of Mutant Strain of Corynebacterium glutamicum CJ7V Strain in which ramA Gene Promoter Is Improved and Substituted and Evaluation of L-Valine Producing Ability of Constructed Strain

In order to identify whether the effect on enhancing the L-valine producing ability is obtained in other L-valine-producing strains belonging to the *Corynebacterium glutamicum,* the wild-type *Corynebacterium glutamicum* ATCC14067 was introduced with one type of modification [ilvN(A42V); Biotechnology and Bioprocess Engineering, June 2014, Volume 19, Issue 3, pp 456-467] to prepare a strain having enhanced L-valine producing ability.

Specifically, genomic DNA of the wild-type *Corynebacterium glutamicum* ATCC14067 strain was extracted using a G-spin Total DNA Extraction Mini Kit (Intron, Cat. No 17045) in accordance with protocols of the kit. PCR was performed using the genomic DNA as a template. In order to construct a vector introducing A42V modification into ilvN gene, gene fragments A and B were obtained using a primer pair of SEQ ID NOS: 25 and 26; and a primer pair of SEQ ID NOS: 27 and 28, respectively. The PCR was performed under the following conditions: denaturation at 94°C for 5 minutes; 25 cycles of denaturation at 94°C for 30 seconds, annealing at 55°C for 30 seconds, and polymerization at 72°C for 60 seconds, and then polymerization at 72°C for 7 minutes.

As a result, polynucleotide fragments A and B both including 537 bp were obtained. A PCR product of 1044 bp (hereinafter, referred to as "modification-introduced fragment 2") was obtained by performing overlapping PCR using the two fragments as templates with SEQ ID NOS: 25 and 26.

After treating the obtained modification-introduced fragment 2 with the restriction enzyme Xbal (New England Biolabs, Beverly, MA), the modification-introduced fragment 2 was ligated to a pDZ vector treated with the same restriction enzyme using a T4 ligase (New England Biolabs, Beverly, MA). After transforming E. *coli* DH5α with the constructed gene, transformed strains were selected in an LB medium containing kanamycin and DNA was obtained therefrom using a DNA-spin plasmid DNA purification kit (iNtRON). The vector to be used to introduce A42V modification of the ilvN gene was named pDZ-ilvN(A42V).

**Table 6**

| Primer | Base sequence | SEQ ID NO: |
|---|---|---|
| Primer 17 | aatttctagaggcagaccctattctatgaagg | SEQ ID NO: 25 |
| Primer 18 | agtgtttcggtctttacagacacgagggac | SEQ ID NO: 26 |
| Primer 19 | gtccctcgtgtctgtaaagaccgaaacact | SEQ ID NO: 27 |
| Primer 20 | aatttctagacgtgggagtgtcactcgcttgg | SEQ ID NO: 28 |

Subsequently, the wild-type *Corynebacterium glutamicum* ATCC14067 was transformed with the pDZ-ilvN(A42V) vector via chromosomal homologous recombination (van der Rest et al., Appl Microbiol Biotechnol 52:541-545, 1999). Strains whose chromosome was inserted with the vector by homologous sequence recombination were selected in a culture medium containing kanamycin (25 mg/f). Then, the gene fragments were amplified by PCR performed on *Corynebacterium glutamicum* transformants in which secondary recombination was completed using SEQ ID NOS: 25 and 26 and strains into which modification was inserted were confirmed by gene sequencing. The recombinant strain was named *Corynebacterium glutamicum* CJ7V.

Finally, the *Corynebacterium glutamicum* CJ7V was transformed with the vectors in the same manner as in Examples 3-1 and 3-2, and the strains were named *Corynebacterium glutamicum* CJ7V-Pm1-ramA, CJ7V-Pm2-ramA, CJ7V-Pm3-ramA and CJ7V-Pcj7-ramA, respectively. For comparison of L-valine producing ability between the constructed strains, the strains were cultured in the same manner as in Example 3-1 above, concentrations of L-valine were analyzed, and the analyzed concentrations of L-valine are shown in Table 7 below.

**Table 7**

| Comparison of L-valine producing ability | | | | |
|---|---|---|---|---|
| Strain | L-valine (g/L) | | | |
| | Batch 1 | Batch 2 | Batch 3 | Average |
| CJ7V | 2.2 | 2.2 | 2.3 | 2.2 |
| CJ7V-Pm1-ramA | 2.8 | 2.7 | 2.7 | 2.7 |
| CJ7V-Pm2-ramA | 2.6 | 2.6 | 2.7 | 2.6 |
| CJ7V-Pm3-ramA | 2.6 | 2.4 | 2.5 | 2.5 |
| CJ7V-Pcj7-ramA | 2.4 | 2.5 | 2.6 | 2.5 |

As shown in Table 7, it was confirmed that the CJ7V-Pm1-ramA, CJ7V-Pm2-ramA and CJ7V-Pm3-ramA strains including the improved promoter had L-valine production increased by about 23%, 18%, and 14%, respectively, which are similar to or higher than L-valine producing ability of the CJ7V-Pcj7-ramA strain substituted with the stronger promoter.

### Example 3-4: Construction of Mutant Strain of Corynebacterium glutamicum CJ8V in which ramA Gene Promoter is Improved and Substituted and Evaluation of L-Valine Producing Ability of Constructed Strain

In order to identify whether the effect on enhancing the L-valine producing ability is obtained in other L-valine-producing strains belonging to the *Corynebacterium glutamicum,* the wild-type *Corynebacterium glutamicum* ATCC13869 was introduced with one type of modification [ilvN(A42V)] in the same manner as the method of Example 3-3 to prepare a strain having L-valine producing ability and the recombinant strain was named *Corynebacterium glutamicum* CJ8V.

Finally, the *Corynebacterium glutamicum* CJ8V was transformed with the vectors in the same manner as the method of Examples 3-1 and 3-2, and the strains were named *Corynebacterium glutamicum* CJ8V-Pm1-ramA, CJ8V-Pm2-ramA, CJ8V-Pm3-ramA and CJ8V-Pcj7-ramA, respectively. For comparison of L-valine producing ability between the constructed strains, the strains were cultured in the same manner as in Example 3-1 above, concentrations of L-valine were analyzed, and the analyzed concentrations of L-valine are shown in Table 8 below.

**Table 8**

| L-Valine producing ability | | | | |
|---|---|---|---|---|
| Strain | L-valine (g/L) | | | |
| | Batch 1 | Batch 2 | Batch 3 | Average |
| CJ8V | 1.9 | 2.0 | 1.9 | 1.9 |
| CJ8V-Pm1-ramA | 2.3 | 2.3 | 2.3 | 2.3 |
| CJ8V-Pm2-ramA | 2.3 | 2.1 | 2.2 | 2.2 |
| CJ8V-Pm3-ramA | 2.0 | 2.1 | 2.2 | 2.1 |
| CJ8V-Pcj7-ramA | 2.1 | 2.0 | 1.9 | 2.0 |

As shown in Table 8, it was confirmed that the CJ8V-Pm1-ramA, CJ8V-Pm2-ramA and CJ8V-Pm3-ramA strains including the improved promoter compared to the CJ8V strain had L-valine production increased by about 21%, 16%, and 10%, respectively, which are similar to or higher than L-valine producing ability of the CJ8V-Pcj7-ramA strain substituted with the stronger promoter.

### Example 4. Construction of Mutant Strain of L-Leucine-producing Corynebacterium glutamicum KCCM11661P and KCCM11662P into which Promoter Modification is Introduced and Evaluation of L-Leucine Producing Ability

*Corynebacterium glutamicum* KCCM11661P and KCCM11662P were transformed with the pDZ-Pm1-ramA, pDZ-Pm2-ramA, pDZ-Pm3-ramA, and pDZ-Pcj7-ramA vectors via chromosomal homologous recombination (van der Rest et al., Appl Microbiol Biotechnol 52:541-545, 1999). Strains whose chromosome was inserted with the vector by homologous sequence recombination were selected in a culture medium containing kanamycin (25 mg/L). Then, PCR was performed on the *Corynebacterium glutamicum* transformants in which secondary recombination was completed using SEQ ID NOS: 9 and 10 and strains in which the ramA promoter was improved and substituted with Pcj7 were confirmed. The recombinant strains were named *Corynebacterium glutamicum* KCCM11661P-Pm1-ramA, KCCM11661P Pm2-ramA, K KCCM11661 P-Pm3-ramA, KCCM11661 P-Pcj7-ramA and KCCM11662P-Pm1-ramA, KCCM11662P Pm2-ramA, K KCCM11662P-Pm3-ramA, and KCCM11662P-Pcj7-ramA, respectively.

The constructed strains were cultured according to the following method and leucine producing ability was compared.

After subculturing each of the strains in a nutrient medium, each of the strains was inoculated onto a 250 mL corner-baffled flask containing 25 mL of a production medium, and cultured while shaking at 30°C for 72 hours at 200 rpm. Then, concentrations of L-leucine were analyzed using HPLC, and the analyzed concentrations of L-leucine are shown in Table 9 below.

### <Nutrient Medium (pH 7.2)>

10 g of glucose, 5 g of meat gravy, 10 g of polypeptone, 2.5 g of sodium chloride, 5 g of yeast extract, 20 g of agar, 2 g of urea (based on 1 L of distilled water)

### <Production Medium (pH 7.0)>

50 g of glucose, 20 g of ammonium sulfate, 20 g of corn steep solid, 1 g of dibasic potassium phosphate, 0.5 g of magnesium sulfate heptahydrate, 100 µg of biotin, 1 mg of thiamine-HCI, and 15 g of calcium carbonate (based on 1 L of distilled water)

**Table 9**

| L-Leucine producing ability | | | | |
|---|---|---|---|---|
| Strain | L-Leucine (g/L) | | | |
| | Batch 1 | Batch 2 | Batch 3 | Average |
| KCCM11661P | 2.8 | 2.6 | 2.7 | 2.7 |
| KCCM11661P-Pm1-ramA | 3.2 | 2.9 | 2.8 | 3.0 |
| KCCM11661PPm2-ramA | 3.0 | 2.8 | 2.9 | 2.9 |
| KCCM11661P-Pm3-ramA | 3.1 | 3.1 | 3.0 | 3.0 |
| KCCM11661P-Pcj7-ramA | 3.2 | 3.1 | 3.1 | 3.1 |
| KCCM11662P | 3.0 | 3.1 | 2.9 | 3.0 |
| KCCM11662P-Pm1-ramA | 3.3 | 3.3 | 3.5 | 3.3 |
| KCCM11662P Pm2-ramA | 3.3 | 3.2 | 3.2 | 3.2 |
| KCCM11662P-Pm3-ramA | 3.2 | 3.5 | 3.3 | 3.3 |
| KCCM11662P-Pcj7-ramA | 3.4 | 3.4 | 3.3 | 3.3 |

As a result, it was confirmed that the KCCM11661P-Pm1-ramA, KCCM11661P Pm2-ramA, and KCCM11661P-Pm3-ramA strains including the improved promoter had L-leucine production increased by 11%, 7%, and 11%, respectively, compared to the KCCM11661P strain which are similar to or higher than L-leucine producing ability of the KCCM11661P-Pcj7-ramA strain substituted with the stronger promoter.

Also, it was confirmed that the KCCM11662P-Pm1-ramA, KCCM11662P Pm2-ramA, and KCCM11662P-Pm3-ramA strains including the improved strain had L-leucine production increased by 10%, 6%, and 10%, respectively, compared to the KCCM11662P strain which are similar to or higher than L-leucine producing ability of the KCCM11662P-Pcj7-ramA strain substituted with the stronger promoter.

### Example 5. Construction of Mutant Strain of L-lsoleucine-producing Corynebacterium glutamicum KCCM11248P in which ramA Gene Promoter is Improved and Substituted and Evaluation of L-lsoleucine Producing Ability

In order to identify whether the effect on enhancing L-isoleucine producing ability is obtained in other L-isoleucine-producing strains belonging to the *Corynebacterium glutamicum,* L-isoleucine-producing *Corynebacterium glutamicum* KCCM11248P strain was transformed with the vectors in the same manner as in Examples 3-1 and 3-2 above and the transformed strains were named *Corynebacterium glutamicum* KCCM11248P-Pm-ramA, KCCM11248P-Pm1-ramA, KCCM11248P-Pm2-ramA, KCCM11248P-Pm3-ramA, and KCCM11248P-Pcj7-ramA, respectively. The KCCM11248P-Pm-ramA, KCCM11248P-Pm1-ramA, KCCM11248P-Pm2-ramA, KCCM11248P-Pm3-ramA, and KCCM11248P-Pcj7-ramA strains were cultured according to the following method and isoleucine producing ability was evaluated.

Each of the strains was inoculated onto a 250 mL corner-baffle flask containing 25 mL of a seed medium and cultured while shaking at 30°C for 20 hours at 200 rpm. Then, 1 mL of the seed medium was inoculated onto a 250 mL corner-baffle flask containing 24 mL of a production medium and cultured while shaking at 30°C for 48 hours at 200 rpm. Compositions of the seed medium and the production medium are as follows.

### <Seed Medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 100 µg of biotin, 1000 µg of thiamine HCl, 2000 µg of calcium pantothenate, and 2000 µg of nicotinamide (based on 1 L of distilled water)

### <Production Medium (pH 7.0)>

50 g of glucose, 12.5 g of (NH₄)₂SO₄, 2.5 g of soy protein, 5 g of corn steep solids, 3 g of urea, 1 g of KH₂PO₄, 0.5 g of MgSO₄·7H₂O, 100 µg of biotin, 1000 µg of thiamine HCl, 2000 µg of calcium pantothenate, 3000 µg of nicotinamide, 30 g of CaCO₃ (based on 1 L of distilled water)

After completion of the culture, concentrations of L-isoleucine were measured by HPLC, the measured concentrations of L-isoleucine are shown in Table 10 below.

**Table 10**

| Strain | L-lsoleucine (g/L) | | | |
|---|---|---|---|---|
| | Batch 1 | Batch 2 | Batch 3 | Average |
| KCCM11248P | 1.6 | 1.3 | 1.4 | 1.43 |
| KCCM11248P-Pm1-ramA | 2.0 | 1.8 | 2.2 | 2.00 |
| KCCM11248P-Pm2-ramA | 1.8 | 2.0 | 1.9 | 1.90 |
| KCCM11248P-Pm3-ramA | 1.7 | 1.8 | 1.6 | 1.70 |
| KCCM11248P-Pcj7-ramA | 1.8 | 1.8 | 1.7 | 1.76 |

As a result, it was confirmed that the KCCM11248P-Pm1-ramA, KCCM11248P-Pm2-ramA, and KCCM11248P-Pm3-ramA strains including the improved promoter had L-isoleucine production increased by 39%, 32%, and 18%, respectively, compared to the KCCM11248P strain which are similar to or higher than L-isoleucine producing ability of the KCCM11248P-Pcj7-ramA strain substituted with the stronger promoter.

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. Therefore, the scope of the disclosure is defined not by the detailed description, but by the claims and their equivalents, and all variations within the scope of the claims and their equivalents are to be construed as being included in the disclosure.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA08-1518 | Accession number given by the
INTERNATIONAL DEPOSITARY AUTHORITY:
KCCM12704P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 27. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 27. 2020.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yurim Bldg., 45, Hongjenae 2ga-gil, Seodaemun-gu, Seoul 03641 Korea Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA08-1519 | Accession number given by the
INTERNATIONAL DEPOSITARY AUTHORITY:
KCCM12705P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 27. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 27. 2020.

¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

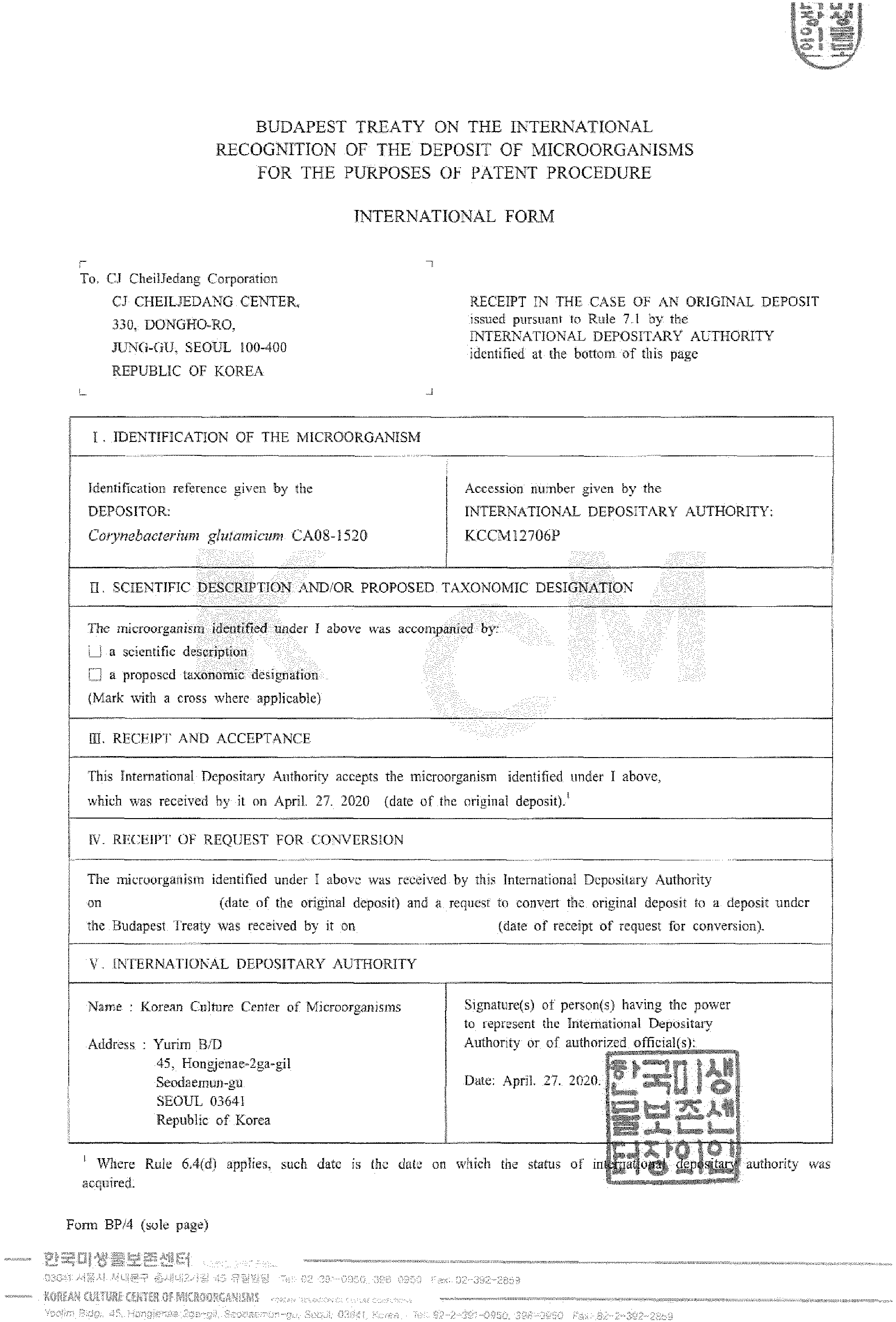
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA08-1520 | Accession number given by the
INTERNATIONAL DEPOSITARY AUTHORITY:
KCCM12706P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on April. 27. 2020 (date of the original deposit).¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 27. 2020.


¹ Where Rule 6.4(d) applies, such date is the date on which the status of international depositary authority was
acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg. 45, Hongjenae 2ga-gil, Seodaemun-gu, Seoul, 03641, Korea Tel: 82-2-391-0950, 396-0950 Fax: 82-2-392-2859

## Claims

1. An L-branched-chain amino acid-producing microorganism having enhanced activity of regulator of acetate metabolism A.

2. The microorganism according to claim 1, wherein the enhanced activity is obtained by: introducing a modification into a gene expression regulatory sequence of regulator of acetate metabolism A; replacing the gene expression regulatory sequence with a sequence improving expression; additionally introducing a modification to the gene to enhance the activity; or a combination thereof.

3. The microorganism according to claim 2, wherein the gene expression regulatory sequence is a promoter.

4. The microorganism according to claim 2, wherein the microorganism comprises a polynucleotide having promoter activity and including substitution of a nucleotide with a different nucleotide at one or more corresponding positions selected from positions 34, 36, 37, 41, and 43 of a nucleotide sequence as set forth in SEQ ID NO: 1.

5. The microorganism according to claim 4, wherein the polynucleotide comprises substitution with T at the 34^{th} nucleotide; substitution with T at the 36^{th} nucleotide; substitution with G at the 37^{th} nucleotide; substitution with T at the 41^{st} nucleotide; substitution with A at the 43^{rd} nucleotide; or a combination thereof, in the nucleotide sequence as set forth in SEQ ID NO: 1.

6. The microorganism according to claim 4, wherein the polynucleotide comprises one nucleotide sequence selected from SEQ ID NOS: 3 to 5.

7. The microorganism according to claim 1, wherein the microorganism is a microorganism of the genus *Corynebacterium.*

8. The microorganism according to claim 7, wherein the microorganism of the genus *Corynebacterium* comprises *Corynebacterium glutamicum.*

9. A method for producing an L-branched-chain amino acid, the method comprising culturing the microorganism according to any one of claims 1 to 8 in a culture medium.

10. The method according to claim 9, further comprising recovering or separating the L-branched-chain amino acid from the culture medium or the microorganism.

11. A polynucleotide having promoter activity and comprising substitution of a nucleotide with a different nucleotide at one or more corresponding positions selected from positions 34, 36, 37, 41, and 43 of a nucleotide sequence as set forth in SEQ ID NO: 1.

12. The polynucleotide according to claim 11, wherein the polynucleotide comprises substitution with T at the 34^{th} nucleotide; substitution with T at the 36^{th} nucleotide; substitution with G at the 37^{th} nucleotide; substitution with T at the 41^{st} nucleotide; substitution with A at the 43^{rd} nucleotide; or a combination thereof, in the nucleotide sequence as set forth in SEQ ID NO: 1.

13. The polynucleotide according to claim 11, wherein the polynucleotide comprises one nucleotide sequence selected from SEQ ID NOS: 3 to 5.
